# EUROPEAN PATENT APPLICATION

(11) **EP 1 496 058 A1**
(43) Date of publication of application: **12.01.2005**
(21) Application number: 03015434.8
(22) Date of filing: 09.07.2003
(51) Int. Cl.: C07D 451/02, A61K 31/46, A61P 29/00

(54) **Novel N-(heterobicycloalkanes)-substituted Indoles or heteroderivatives thereof**

(71) Applicant: Boehringer Ingelheim Pharma GmbH & Co.KG, 55216 Ingelheim am Rhein (DE)
(72) Inventor: Anderskewitz, Ralf Dr., 88471 Laupheim (DE); Martyres, Domnic Dr., 88400 Biberach (DE); Dollinger, Horst Dr., 88433 Schemmerhofen (DE); Pouzet, Pascale Dr., 88400 Biberach (DE); Birke, Franz Dr., 55218 Ingelheim (DE); Bouyssou, Thierry Dr., 88487 Mietingen (DE)

(57) **Abstract**

Use of a compound of general formula 1 for manufacturing a medicament to provide agonists or antagonists of CCR-3, or pharmaceutically acceptable salts thereof, in particular compounds of formula 1: 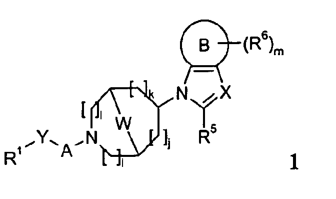 wherein R¹, R⁵, R⁶, A, B, X, W, Z, i, j, k, l and m are defined as in the description and claims.

It is another object of the present invention to provide pharmaceutical compositions comprising a pharmaceutically acceptable carrier and a therapeutically effective amount of at least one of the compounds of the present invention or a pharmaceutically acceptable salt thereof.

## Description

### BACKGROUND OF THE INVENTION

### TECHNICAL FIELD

This invention relates generally to novel tropane-derivatives and their use as modulators of chemokine receptor activity, pharmaceutical compositions containing the same, and methods of using the same as agents for treatment and prevention of inflammatory diseases such as asthma and allergic diseases, as well as autoimmune pathologies such as rheumatoid arthritis and atherosclerosis.

### BACKGROUND INFORMATION

Chemokines are chemotactic cytokines, of molecular weight 6-15 kDa, that are released by a wide variety of cells to attract and activate, among other cell types, macrophages, T and B lymphocytes, eosinophils, basophils and neutrophils (reviewed in Luster, New Eng. J Med., 338, 436-445 (1998) and Rollins, Blood, 90,909-928 (1997)).

There are two major classes of chemokines, CXC and CC, depending on whether the first two cysteines in the amino acid sequence are separated by a single amino acid (CXC) or are adjacent (CC). The CXC chemokines, such as interleukin-8 (IL-8), neutrophil-activating protein-2 (NAP2) and melanoma growth stimulatory activity protein (MGSA) are chemotactic primarily for neutrophils and T lymphocytes, whereas the CC chemokines, such as RANTES, MIP-la, MIP-1 (3, the monocyte chemotactic proteins (MCP-1, MCP-2, MCP-3, MCP-4, and MCP-5) and the eotaxins (-1,-2, and-3) are chemotactic for, among other cell types, macrophages, T lymphocytes, eosinophils, dendritic cells, and basophils. There also exist the chemokines lymphotactin-1, lymphotactin-2 (both C chemokines), and fractalkine (a CXXXC chemokine) that do not fall into either of the major chemokine subfamilies.

The chemokines bind to specific cell-surface receptors belonging to the family of G-protein-coupled seventransmembrane-domain proteins (reviewed in Horuk, Trends Pharm. Sci., 15,159-165 (1994)) which are termed "chemokine receptors." On binding their cognate ligands, chemokine receptors transduce an intracellular signal through the associated trimeric G proteins, resulting in, among other responses, a rapid increase in intracellular calcium concentration, changes in cell shape, increased expression of cellular adhesion molecules, degranulation, and promotion of cell migration. There are at least ten human chemokine receptors that bind or respond to CC chemokines with the following characteristic patterns: CCR1 (or"CKR-1"or"CC-CKR-1") [MIP-1a, MCP-3, MCP-4, RANTES] (Ben-Barruch, et al., Cell, 72,415-425 (1993), Luster, New Eng. J. Med., 338,436-445 (1998)); CCR-2A and CCR-2B (or "CKR-2A"/"CKR-2B"or"CC-CKR-2A"/"CC-CKR-2B") [MCP-1, MCP2, MCP-3, MCP-4, MCP-5] (Charo et al., Proc. Natl. Acad. Sci. USA, 91,2752-2756 (1994), Luster, New Eng. J. Med., 338,436-445 (1998)); CCR-3 (or"CKR-3"or"CC-CKR-3") [eotaxin-1, eotaxin-2, RANTES, MCP-3, MCP-4] (Combadiere, et al., J. Biol. Chem., 270,16491-16494 (1995), Luster, New Eng. J. Med., 338,436-445 (1998)); CCR-4 (or"CKR-4" or"CC-CKR-4") [TARC, MIP-la, RANTES, MCP-1] (Power et al., J. Biol. Chem., 270,19495-19500 (1995), Luster, New Eng. J. Med., 338,436-445 (1998)); CCR-5 (or"CKR-5"OR"CCCKR-5") [MIP-la, RANTES, MIP-lp] (Sanson, et al., Biochemistry, 35,3362-3367 (1996)); CCR-6 (or"CKR-6"or "CC-CKR-6") [LARC] (Baba et al., J. Biol. Chem., 272, 14893-14898 (1997)); CCR-7 (or"CKR-7"or"CC-CKR-7") [ELC] (Yoshie et al., J. Leukoc. Biol. 62,634-644 (1997)); CCR-8 (or"CKR-8"or"CC-CKR-8") [1-309, TARC, MIP-1p] (Napolitano et al., J. Immunol., 157,2759-2763 (1996), Bernardini et al., Eur. J. Immunol., 28,582-588 (1998)); and CCR-10 (or"CKR-10"or"CC-CKR-10") [MCP-1, MCP-3] (Bonini et al, DNA and Cell Biol., 16,1249-1256 (1997)).

In addition to the mammalian chemokine receptors, mammalian cytomegaloviruses, herpes viruses and poxviruses have been shown to express, in infected cells, proteins with the binding properties of chemokine receptors (reviewed by Wells and Schwartz, Curr. Opin. Biotech., 8, 741-748 (1997)). Human CC chemokines, such as RANTES and MCP-3, can cause rapid mobilization of calcium via these virally encoded receptors. Receptor expression may be permissive for infection by allowing for the subversion of normal immune system surveillance and response to infection. Additionally, human chemokine receptors, such as CXCR-4, CCR-2, CCR-3, CCR-5 and CCR-8, can act as coreceptors for the infection of mammalian cells by microbes as with, for example, the human immunodeficiency viruses (HIV).

Chemokine receptors have been implicated as being important mediators of inflammatory, infectious, and immunoregulatory disorders and diseases, including asthma and allergic diseases, as well as autoimmune pathologies such as rheumatoid arthritis and atherosclerosis. For example, the chemokine receptor CCR-3 plays a pivotal role in attracting eosinophils to sites of allergic inflammation and in subsequently activating these cells. The chemokine ligands for CCR-3 induce a rapid increase in intracellular calcium concentration, increased expression of cellular adhesion molecules, cellular degranulation, and the promotion of eosinophil migration. Accordingly, agents which modulate chemokine receptors would be useful in such disorders and diseases. In addition, agents which modulate chemokine receptors would also be useful in infectious diseases such as by blocking infection of CCR-3 expressing cells by HIV or in preventing the manipulation of immune cellular responses by viruses such as cytomegaloviruses.

### BACKGROUND ART

- WO 02 096906 discloses Azabicyclo[3,2,1]octanes for the modulation of the 5-HT_{1A} receptor for the treatment of i.e. depression or fear.
- WO 01 66525 discloses substituted benzimidazoles or benzotriazoles for the modulation of the CCR5 receptor for the treatment i.e. HIV1, inflammatory or immunoregulatory. One can also fmd in the description the assumption that the disclosed compounds have potential in the treatment of asthma or COPD.

### BRIEF SUMMARY OF THE INVENTION

Accordingly, one object of the present invention is to provide agonists or antagonists of CCR-3, or pharmaceutically acceptable salts thereof, in particular compounds of formula 1, 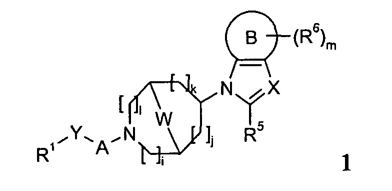 wherein R¹, R⁵, R⁶, A, B, X, W, i, j, k, l and m are defined as below.

It is another object of the present invention to provide pharmaceutical compositions comprising a pharmaceutically acceptable carrier and a therapeutically effective amount of at least one of the compounds of the present invention or a pharmaceutically acceptable salt thereof.

These and other objects, which will become apparent during the following detailed description.

### DETAILED DESCRIPTION OF THE INVENTION

The present invention relates to the use of a compound of general formula 1 for manufacturing a medicament for the prevention and/or treatment of diseases wherein the activity of a CCR-3 receptor is involved. 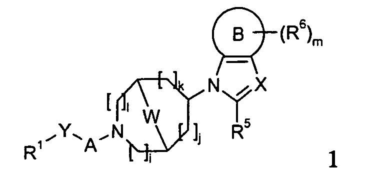 wherein
- R¹ is: aryl, het or a annelated species thereof, wherein the annelated species comprises aryl-het-, het-aryl- or het-het-annelations, each of said aryl or het may be substituted with one, two or three R²;
- R² are: each independently -C₁₋₆-alkyl, -C₃₋₆-cycloalkyl, -C₁₋₆-haloalkyl, -C₁₋₆-aralkyl, halogen, -CN, -COOR³, -COR³, -CONR³R⁴, -NR³R⁴, -NR³COR⁴, -NR³SO₂R⁴, -OR³, -NO₂, -SR³, -SOR³, -SO₂R³ or -SO₂NR³R⁴;
- R³ is: H, -C₁₋₆-alkyl, -C₃₋₈-cycloalkyl or (-C₃₋₈-cycloalkyl)-C₁₋₆-alkyl;
- R⁴ is: H, -C₁₋₆-alkyl, -C₃₋₈-cycloalkyl or (-C₃₋₈-cycloalkyl)-C₁₋₆-alkyl or
- R³ and R⁴: together with the interjacent nitrogen atom or -N-SO₂- group form an optionally substituted nitrogen containing heterocyclic 3 to 8 membered ring.
- R⁵ is: -C₁₋₆-alkyl, -C₁₋₆-alkoxy, -C₁₋₆-acyloxy, -C₁₋₆-aralkyl, -C₃₋₆-cycloalkyl, (-C₃₋₆-cycloalkyl)-C₁₋₆-alkyl, -C₁₋₆-haloalkyl, -C₁₋₆-haloalkoxy, -C₁₋₆-haloalkylthio, -C₁₋₆-thioalkyl, halogen, -NO₂, -OH, -CN;
- R⁶ are: each independently -C₁₋₆-alkyl, -C₁₋₆-alkoxy, -C₁₋₆-acyloxy, -C₁₋₆-aralkyl, -C₃₋₆-cycloalkyl, -C₁₋₆-haloalkyl, -C₁₋₆-thioalkyl, halogen, -OR³, -SR³, -CN, -NO₂, -COOR^{3,} -COR^{3,} -CONR³R⁴, -NR³R⁴, -NR³COR⁴, -NR³SO₂R⁴, -SOR³, -SO₂R³, -SO₂NR³R⁴, aryl or het;
- A is: -C₂₋₈-alkylene, optionally substituted with halogen or -OH;
- B is: aryl or het;
- X is: N, CH or CR⁶;
- Y is: -CF₂-, -NR⁴-, -O-, -S(O)ₙ-;
- W is: -C₁₋₃-alkylene, -CH₂-Z-CH₂-;
- Z is: -O-, -S-, -NR⁴-;
- i, j, k, l are: each independently 0, 1 or 2, wherein 2 ≤ i+ j + k + l ≤ 4;
- n is: 0, 1 or 2;
- m is: 0, 1, 2, 3 or 4.

The present invention relates to the use of the compounds of general formula 1 and to the novel compounds of formula **1** in which Y is S or CF₂.

The compounds herein described may have asymmetric centres. Compounds of the present invention containing an asymmetrically substituted atom may be isolated in optically active or racemic forms. It is well known in the art how to prepare optically active forms, such as by resolution of racemic forms or by synthesis from optically active starting materials. Many geometric isomers of olefins, C=N double bonds, and the like can also be present in the compounds described herein, and all such stable isomers are contemplated in the present invention. Cis and trans geometric isomers of the compounds of the present invention are described and may be isolated as a mixture of isomers or as separated isomeric forms. All chiral, diastereomeric, racemic forms and all geometric isomeric forms of a structure are intended, unless the specific stereochemistry or isomeric form is specifically indicated.

### USED TERMS AND DEFINITIONS

Terms not specifically defined herein should be given the meanings that would be given to them by one of skill in the art in light of the disclosure and the context. As used in the specification, however, unless specified to the contrary, the following terms have the meaning indicated and the following conventions are adhered to.
In the groups, radicals, or moieties defined below, the number of carbon atoms is often specified preceding the group, for example, -C₁₋₆ alkyl means an alkyl group or radical having 1 to 6 carbon atoms. In general, for groups comprising two or more subgroups, the last named group is the radical attachment point, for example, "thioalkyl" means a monovalent radical of the formula HS-Alk-. Unless otherwise specified below, conventional definitions of terms control and conventional stable atom valences are presumed and achieved in all formulas and groups.

In general, all tautomeric forms and isomeric forms and mixtures, whether individual geometric isomers or optical isomers or racemic or non-racemic mixtures of isomers, of a chemical structure or compound is intended, unless the specific stereochemistry or isomeric form is specifically indicated in the compound name or structure.

The term "substituted" as used herein, means that any one or more hydrogens on the designated atom is replaced with a selection from the indicated group, provided that the designated atom's normal valence is not exceeded, and that the substitution results in a stable compound.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are preferred. Lists of suitable salts are found in Remingto which release an active parent drug of the present invention in vivo when such prodrug is administered to a mammalian subject. Prodrugs the present invention are prepared by modifying functional groups present in the compound in such a way that the modifications are cleaved, either in routine manipulation or in vivo, to the parent compound. Prodrugs include compounds of the present invention wherein a hydroxy, amino, or sulfhydryl group is bonded to any group that, when the prodrug of the present invention is administered to a mammalian subject, it cleaves to form a free hydroxyl, free amino, or free sulfhydryl group, respectively. Examples of prodrugs include, but are not limited to, acetate, formate and benzoate derivatives of alcohol and amine functional groups in the compounds of the present invention.

The term "aryl" as used herein, either alone or in combination with another substituent, means either an aromatic monocyclic system or aromatic multicyclic systems containing carbon atoms. For example, aryl includes a phenyl or a naphthyl ring system, wherein aryl means generally an aromatic system, for example phenyl.

The term "het" as used herein, either alone or in combination with another substituent, means a monovalent substituent derived by removal of a hydrogen from a five-, six- or seven-membered saturated or unsaturated (including aromatic) heterocycle containing carbon atoms and one, two, three or four ring heteroatoms selected from nitrogen, oxygen and sulfur. Examples of suitable heterocycles include: tetrahydrofuran, thiophene, diazepine, isoxazole, piperidine, dioxane, morpholine, piperazine or

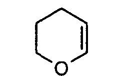

Although generally covered under the term "het", the term "heteroaryl" as used herein precisely defines an unsaturated heterocycle for which the double bonds form an aromatic system. Suitable example of heteroaromatic system include: pyridine, pyrimidine,

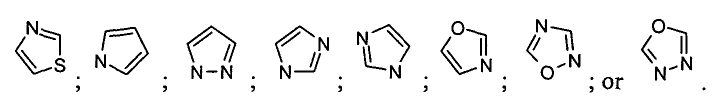

The term "annelated species of aryl or het" as used herein, either alone or in combination with another substituent wherein the annelated species presents as a aryl-het (a), a het-aryl (b) or a het-het (c) annelation means a monovalent substituent derived by removal of one hydrogen from
a) an aromatic monocyclic system or aromatic multicyclic systems containing carbon atoms, which is annelated to a five-, six- or seven-membered saturated or unsaturated (including aromatic) heterocycle containing carbon atoms and one, two, three or four ring heteroatoms selected from nitrogen, oxygen and sulfur or
b) a five-, six-, or seven-membered saturated or unsaturated (including aromatic) heterocycle containing carbon atoms and one, two, three or four ring heteroatoms selected from nitrogen, oxygen and sulfur, which is annelated to an aromatic monocyclic system or aromatic multicyclic systems containing carbon atoms or
c) a five-, six-, or seven-membered saturated or unsaturated (including aromatic) heterocycle containing carbon atoms and one, two, three or four ring heteroatoms selected from nitrogen, oxygen and sulfur, which is annelated to a five-, six-, or seven-membered saturated or unsaturated (including aromatic) heterocycle containing carbon atoms and one, two, three or four ring heteroatoms selected from nitrogen, oxygen and sulfur.
Suitable examples of a annelated species of aryl or het include: quinolinyl, 1-indoyl, 3-indoyl, 5-indoyl, 6-indoyl, indolizinyl, benzimidazyl or purinyl.

The term "halogen" as used herein means a halogen substituent selected from fluoro, chloro, bromo or iodo.

The term "-C₁₋₆-alkyl" as used herein, either alone or in combination with another substituent, means acyclic, straight or branched chain alkyl substituents containing from one to six carbon atoms and includes, for example, methyl, ethyl, propyl, butyl, hexyl, 1-methylethyl, 1-methylpropyl, 2-methylpropyl or 1,1-dimethylethyl.

The term "-C₃₋₈-cycloalkyl" as used herein, either alone or in combination with another substituent, means a cycloalkyl substituent containing from three to six carbon atoms and includes cyclopropyl, cyclobutyl, cyclopentyl or cyclohexyl.

The term "-C₁₋₆-haloalkyl" as used herein, either alone or in combination with another substituent, means acyclic, straight or branched chain alkyl substituents containing up to six carbon atoms having one or more hydrogens substituted for a halogen selected from bromo, chloro, fluoro or iodo. Accordingly "-C₂-₆-haloalkyl" has the same meaning with exception that the chain contains two to six carbon atoms. Preferably the term -C₁₋₆-haloalkyl represents -C₁₋₆-fluoroalkyl such as 2-fluorethyl, 2,2,2-trifluorethyl or perfluorethyl.

The term "-C₁₋₆-alkoxy" as used herein, either alone or in combination with another substituent, means the substituent -C₁₋₆-alkyl-O- wherein alkyl is as defined above containing up to six carbon atoms. Alkoxy includes methoxy, ethoxy, propoxy, 1-methylethoxy, butoxy or 1,1-dimethylethoxy. The latter substituent is known commonly as t-butoxy.

The term "-C₁₋₆-acyloxy" as used herein, either alone or in combination with another substituent, means the substituent -C₁₋₆-alkyl-(CO)O- wherein alkyl is as defined above containing up to six carbon atoms. Acyloxy includes MeCOO-, EtCOO-, ⁿPrCOO-, ⁱPrCOO-, ⁿBuCOO-, ^{sec}BuCOO- or ^{tert}BuCOO-.

The term "-C₁₋₆-aralkyl" as used herein, either alone or in combination with another substituent, means the substituent -Aryl-C₁₋₆-alkyl- wherein alkyl is as defined above containing up to six carbon atoms. Aralkyl includes benzyl, phenylethyl, phenylpropyl, 1-phenyl-1-methylethyl, phenylbutyl or 1-phenyl-1,1-dimethylethoxy.

The term "-C₁₋₆-thioalkyl" as used herein, either alone or in combination with another substituent, means acyclic, straight or branched chain alkyl substituents containing up to six carbon atoms and a thiol (HS) group as a substituent. An example of a thioalkyl group is a thiopropyl, e.g., HS-CH₂CH₂CH₂-.

The term "-C₂₋₈-alkylene" as used herein means a divalent alkyl substituent derived by the removal of one hydrogen atom from each end of a saturated straight or branched chain aliphatic hydrocarbon containing from two to eight carbon atoms and includes, for example, CH₂CH₂C(CH₃)₂CH₂CH₂-. Accordingly "-C₁₋₃-alkylene" has the same meaning with exception that the chain contains one to three carbon atoms.

### PREFERRED EMBODIMENTS

The compounds of the instant application are useful for manufacturing a medicament for the prevention and/or treatment of diseases wherein the activity of a CCR-3-receptor is involved.

Preferred is the manufacturing of a medicament for the prevention and/or treatment of a wide variety of inflammatory, infectious, and immunoregulatory disorders and diseases, including asthma and allergic diseases, infection by pathogenic microbes (which, by definition, includes viruses), as well as autoimmune pathologies such as the rheumatoid arthritis and atherosclerosis.

Most preferred is the manufacturing of a medicament for the prevention and/or treatment of e.g. inflammatory or allergic diseases and conditions, including respiratory allergic diseases such as asthma, allergic rhinitis, hypersensitivity lung diseases, hypersensitivity pneumonitis, eosinophilic cellulitis (e. g., Well's syndrome), eosinophilic pneumonias (e. g., Loeffler's syndrome, chronic eosinophilic pneumonia), eosinophilic fasciitis (e. g., Shulman's syndrome), delayed-type hypersensitivity, interstitial lung diseases (ILD) (e. g., idiopathic pulmonary fibrosis, or ILD associated with rheumatoid arthritis, systemic lupus erythematosus, ankylosing spondylitis, systemic sclerosis, Sjogren's syndrome, polymyositis or dermatomyositis); systemic anaphylaxis or hypersensitivity responses, drug allergies (e. g., to penicillin, cephalosporins), eosinophilia-myalgia syndrome due to the ingestion of contaminated tryptophan, insect sting allergies; autoimmune diseases, such as rheumatoid arthritis, psoriatic arthritis, multiple sclerosis, systemic lupus erythematosus, myasthenia gravis, juvenile onset diabetes; glomerulonephritis, autoimmune thyroiditis, Behcet's disease; graft rejection (e. g., in transplantation), including allograft rejection or graftversus-host disease; inflammatory bowel diseases, such as Crohn's disease and ulcerative colitis; spondyloarthropathies; scleroderma; psoriasis (including Tcell mediated psoriasis) and inflammatory dermatoses such as an dermatitis, eczema, atopic dermatitis, allergic contact dermatitis, urticaria; vasculitis (e. g., necrotizing, cutaneous, and hypersensitivity vasculitis); eosinophilic myositis, eosinophilic fasciitis; cancers with leukocyte infiltration of the skin or organs.

Preferred are compounds of general formula 1a, wherein R¹, R⁵, R⁶, A, B, X, W, i, j, k, l and m are defined as above.

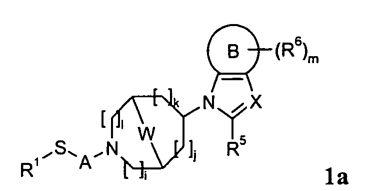

Particularly preferred are compounds of general formula **1b**, wherein R¹, R⁵, R⁶, A, B, X, Y and m are defined as above.

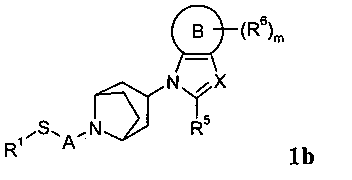

Most preferred are compounds of general formula **1c**, wherein R⁵ is defined as above and R² is -CF₃ or halogen; and Hal is halogen.

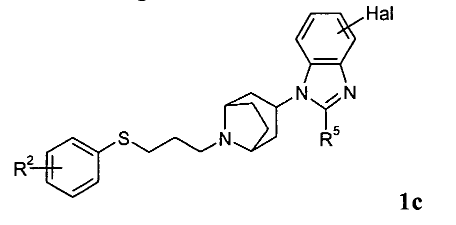

Also preferred are compounds of general formula **1d,** wherein R¹, R⁵, R⁶, A, B, X, W, i, j, k, l and m are defined as in above.

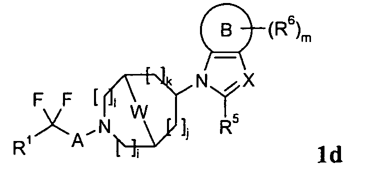

Particularly preferred are compounds of general formula **1e**, wherein R¹, R⁵, R⁶, A, B, X and m are defined as above.

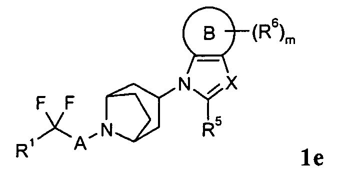

Furthermore preferred are compounds of the general formula **1, 1a, 1b, 1d** or **1e** wherein X has the meaning of a nitrogen atom.

More preferred are the compounds wherein the nitrogen containing heterocyclic link between A and the hetero-bi-cyclus has the meaning of a nortropane.

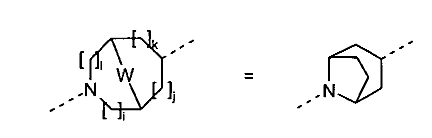

The dotted line in the figure above has the meaning of a bond to the groups like described above in formulae **1, 1a, 1b** or **1d.**

Most preferred are compounds of the general formula **1, 1a, 1b, 1c, 1d** or **1e** wherein:
■ R¹ is aryl or het, both optionally substituted with one, two or three R²; more preferred R¹ is phenyl, optionally substituted with one, two or three R², or
■ R² is -CF₃ or halogen, in particular fluorine; or
■ R⁵ is -C₁₋₆-acyloxy, -C₁₋₆-aralkyl, -C₃₋₆-cycloalkyl, (-C₃₋₆-cycloalkyl)-C₁₋₆-alkyl, - C₂₋₆-haloalkyl or -C₁₋₆-thioalkyl; in particular -C₃₋₆-cycloalkyl or -C₁₋₆-fluoroalkyl; or
■ R⁶ is preferably halogen, in particular fluorine; or
■ A is -CH₂-CH₂-CH₂-; or
■ B is phenyl: or
■ m is 1 or 2.

The compounds of formula **1a, 1b, 1c, 1d** or **1e** can be prepared using the reactions and techniques described below. The reactions are performed in a solvent appropriate to the reagents and materials employed and suitable for the transformations being effected. It will be understood by those skilled in the art of organic synthesis that the functionality present on the molecule should be consistent with the transformations proposed. This will sometimes require a judgment to modify the order of the synthetic steps or to select one particular process scheme over another in order to obtain a desired compound of the invention. It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene and Wuts (Protective Groups In Organic Synthesis, Wiley and Sons, 1991).

### PREPARATION

Compounds of the general formula 1, wherein X has the meaning of a nitrogen atom are prepared by adding a ring B, substituted at least by one nitro-function and a suitable leaving group (LG = i. e. F, Cl) in *ortho-*position*.*

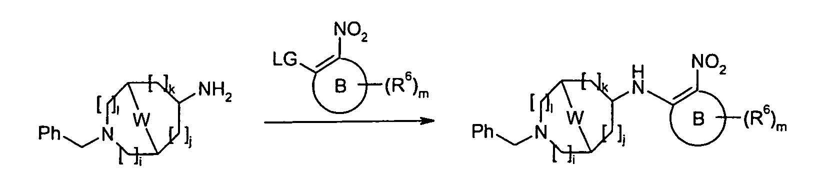

After coupling reaction, the nitro function is reduced by Hydrogen in presence of a Platinum / Charcoal catalyst to a free amine group.

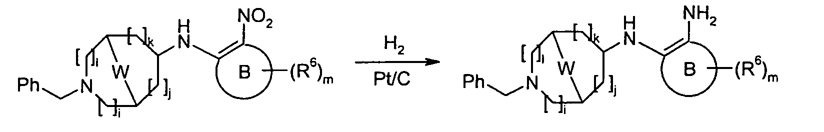

In the next step of the reaction, the resulting amine function is coupled with a acid in presence of PPA to a peptide function.

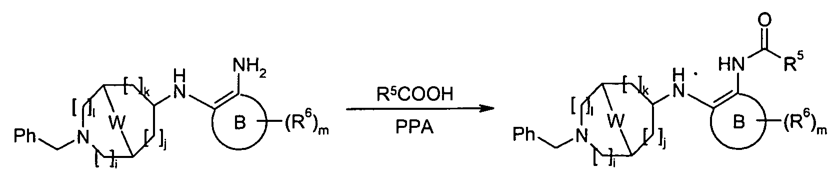

After *N*-acylating reaction, ring closing in presence of a acid is promoted, 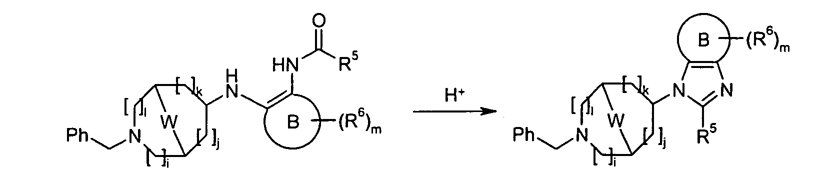 wherein the whole process R⁵, R⁶, B, W, i, j, k, l and m are defined as above.

Alter this, the protecting benzyl group of the 8-aza-bicyclus is removed and preferably, the compounds of general formula 1 are prepared by reaction of a compound of general formula 2 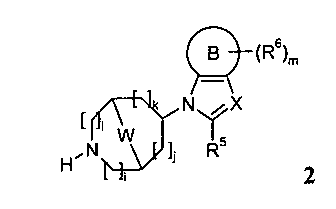 with a compound of the general formula 3. 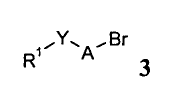 wherein R¹, R⁵, R⁶, A, B, X, W, i, j, k, l and m are defined as for general formula 1 above.

As will be appreciated by one of skill in the art, numerous modifications and variations of the present invention are possible in light of the above teachings. It is therefore to be understood that within the scope of the appended claims, the invention may be practiced otherwise than as specifically described herein.

### EXAMPLE 1

8-Benzyl-8-aza-bicyclo[3.2.1]oct-3-ylamine was obtained according to literature (Dostert et al, Eur. J. Med. Chem., 19, 105-110 (1984)).

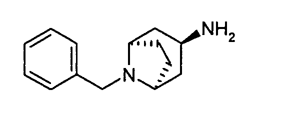

### EXAMPLE 2

### N-(8-Benzyl-8-aza-bicyclo[3.2.1]oct-3-yl)-4-fluoro-benzene-1-nitro-2-diamin

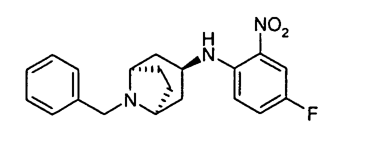

10 g 2-Chloro-5-fluoro-nitrobenzene and 23.7 g 8-benzyl-8-aza-bicyclo[3.2.1]oct-3-yl-amine are stirred 15 h at 150°C. After cooling the residue is treated with ethyl acetate and water, and the organic layer is extracted 3x with water, dried over MgSO₄ and the solvent evaporated. The residue is purified by flash chromatography with CH₂Cl₂/MeOH 95:5 and recrystallized from MeOH. Yield: 11.5 g of orange crystals.
This reaction can also be performed with 2,5-difluoro-nitrobenzene.

### EXAMPLE 3

### N-(8-Benzyl-8-aza-bicyclo[3.2.1]oct-3-yl)-4-fluoro-benzene-1,2-diamine

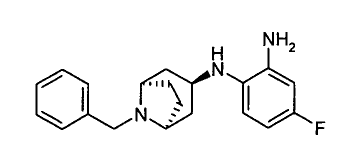

4 g *N*-(8-Benzyl-8-aza-bicyclo[3.2.1]oct-3-yl)-4-fluoro-benzene-1-nitro-2-diamine is dissolved in 100 ml THF and 50 ml MeOH and hydrogenated with 10 % Pt/C for 2 h. The resulting 3.8 g yellow-brown oil is directly used for the next reaction.

### EXAMPLE 4

### Cyclopropanecarboxylic acid [2-(8-benzyl-8-aza-bicyclo[3.2.1]oct-3-ylamino)-5-fluoro-phenyl]-amide

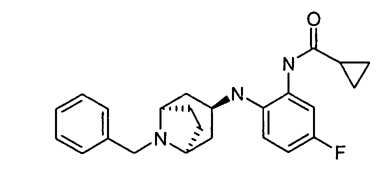

1.9 g *N*-(8-Benzyl-8-aza-bicyclo[3.2.1]oct-3-yl)-4-fluoro-benzene-1,2-diamine and 0.6 g triethylamine are dissolved in 20 ml THF and treated drop wise with cyclopropanoyl chloride at 10-15°C. The mixture is stirred for 2 h, treated with ethyl acetate and water. Thereafter the organic layer is extracted 2x with water, dried over MgSO₄ and the solvent evaporated. The residue is purified by flash chromatography with CH₂Cl₂/MeOH (+ NH₃) yielding 1.25 g brownish oil, which crystallizes during standing.

### EXAMPLE 5

### 1-(8-Benzyl-8-aza-bicyclo[3.2.1]oct-3-yl)-2-cyclopropyl-fluoro-1H-benzoimidazole

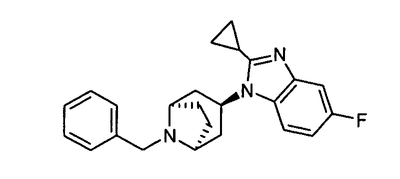

1.25 g Cyclopropanecarboxylic acid [2-(8-benzyl-8-aza-bicyclo[3.2.1]oct-3-ylamino)-5-fluoro-phenyl]-amide is dissolved in 30 ml glacial acetic acid and heated under reflux for 4 h. After cooling water is added to a volume of 200 ml and the solution is treated with K₂CO₃ solution until alkaline reaction. After extraction with ethyl acetate, the organic layer is treated with saturated NaCl solution, the organic layer is dried over MgSO₄ and the solvent evaporated. Yield: 1.0 g of a brownish oil.

### EXAMPLE 6

### 1-(8-Aza-bicyclo[3.2.1]oct-3-yl)-2-cyclopropyl-fluoro-1H-benzoimidazole

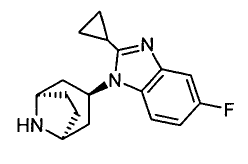

1 g 1-(8-Benzyl-8-aza-bicyclo[3.2.1]oct-3-yl)-2-cyclopropyl-fluoro-1H-benzoimidazole is dissolved in 30 ml MeOH and hydrogenated with 10 % Pd/C at 60 psi and 50°C for 6 h. Tue catalyst is removed and the solvent evaporated. Yield: 0.75 g of a yellow-brown oil.

### EXAMPLE 7

### 1-(3-Bromo-propylsulfanyl)-4-fluoro-benzene

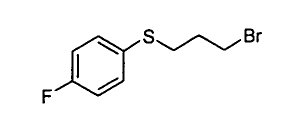

5.3 ml 4-Fluoro-benzothiole, 15.3 ml 1,3-dibromo-propane, 14 g K₂CO₃ and 100 ml CH₃CN are refluxed for 3 h. The insoluble salts are removed, the solvent is evaporated and the residue dissolved in 70 ml ethyl acetate. The solution is extracted with diluted NaOH and water, dried and the solvent is evaporated. The residue is distilled. The fraction between 150°C and 155°C was collected to yield 8 g of pure product.

### EXAMPLE 8

### 1-(4-Bromo-1,1-difluoro-butyl)-4-fluoro-benzene

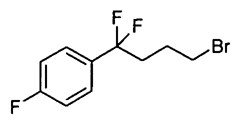

### EXAMPLE 9

### 2-Cyclopropyl-5-fluoro-1-{8-[3-(4-fluoro-phenylsulfanyl)-propyl]-8-aza-bicyclo[3.2.1]oct-3-yl} 1H-benzoimidazole

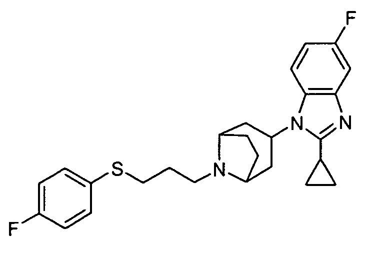

0.74 g 1-(8-Aza-bicyclo[3,2,1]oct-3-yl)-2-cyclopropyl-5-fluoro-1H-benzimidazole, 0.75 g 1-(3-bromo-propylthio)-4-fluoro-benzene, and 0.83 g K₂CO₃ is suspended in 10 ml of DMF. After stirring for 4 h at 100°C, the mixture is allowed to cool down to room temperature, then it is diluted with 300 ml water and extracted twice with ethyl acetate. The separated organic phase is extracted twice with water and dried with MgSO₄. After removal of the solvent the residue is separated over a column (silica gel; CH₂Cl₂/Methanol 98:2). Thereafter the fraction containing the product is freed from solvent, resolved in acetone / ethanolic hydrochloric acid and diluted with diethyl ether. The collected precipitation corresponds to the product in its dihydrochlorid salt form in a yield of 0.1 g.

¹H-NMR (DMSO-d₆): δ= 1.29 ppm (m, 4H), 2.10-2.33 (m, 8H), 2.85 (m, 1H), 3.00-3.12 (m, 6H), 4.22 (br., 2H), 5.12 (hept, 1H), 7.15-7.29 (m, 3H), 7.48 (m, 3H), 8.87 (dd, 1H), 11.69 (br., 1H).

According to example 9 the following examples 10 -15 are prepared

### EXAMPLE 10

### 2-Ethyl-5-fluoro-1-{8-[3-(4-fluoro-phenylsulfanyl)-propyl]-8-aza-bicyclo[3,2,1]oct-3-yl}-1H-benzoimidazole

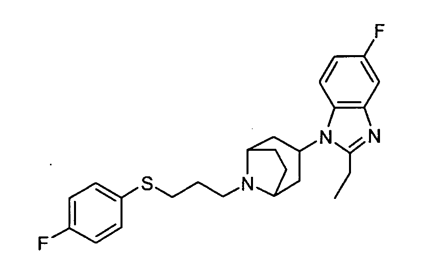

Melting Point: 127-128°C (Compound * 2 HCl). Phase Transitions at 40-45°C and 92-94°C. ¹H-NMR (CDCl₃): 1.42 ppm (t, 3H), 2.11-2.35 (m, 8H), 2.97-3.14 (m, 6H), 3.38 (q, 2H), 4.22 (br., 2H), 4.80 (hept, 1H), 7.20 (dt, 2H), 7.33 (m, 1H), 7.49 (m, 2H), 7.64 (dd, 1H), 9.11 (dd, 1H), 11.78 (br., 1H). ¹³C-NMR (DMSO-d₆): δ= 10.07, 19.75, 23.83, 23.84, 30.70, 32.00, 48.01, 49.76, 115.23, 115.25, 131.05, 157.32 ppm.
MS: [M+H]⁺: 442. [M+HCO₂]⁻: 476.

### EXAMPLE 11

### 5-Fluoro-1-{8-[3-(4-fluoro-phenylsulfanyl)-propyl]-8-aza-bicyclo[3,2,1]oct-3-yl}-2-methyl-1H-benzoimidazole

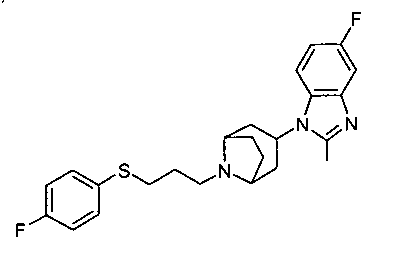

Melting Point: 240-241°C (Compound * 2 HCl). ¹H-NMR (DMSO-d₆):δ= 2.16-2.30 ppm (m, 8H), 2.97-3.15 (m, 6H), 3.00 (s, 3H), 4.24 (br., 2H), 4.82 (hept, 1H), 7.20 (dt, 2H), 7.33 (m, 1H), 7.49 (m, 2H), 7.65 (dd, 1H), 9.18 (dd, 1H), 11.89 (br., 1H). MS: [M+H]⁺: 428.

### EXAMPLE 12

### 5-Fluoro-1-{8-[3-(4-fluoro-phenylsulfanyl)-propyl]-8-aza-bicyclo[3,2,1]oct-3-yl}-2-(2,2,2-trifluoro-ethyl)-1H-benzoimidazole

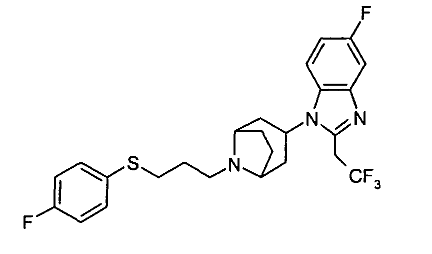

### EXAMPLE 13

### 1-{8-[4,4-Difluoro-4-(4-fluoro-phenyl)-butyl]-8-aza-bicyclo[3.2.1]oct-3-yl}-5-fluoro-2-(2,2,2-trifluoro-ethyl)-1H-benzoimidazole

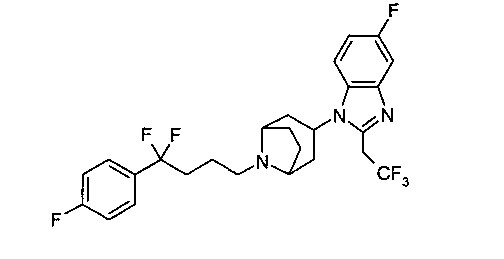

### EXAMPLE 14

### 1-{8-[4,4-Difluoro-4-(4-fluoro-phenyl)-butyl]-8-aza-bicyclo[3.2.1]oct-3-yl}-2-ethyl-5,6-difluoro-1H-benzoimidazole

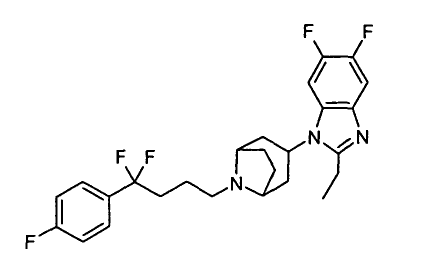

### EXAMPLE 15

### 2-Ethyl-5,6-difluoro-1-{8-[3-(4-fluoro-phenylsulfanyl)-propyl]-8-aza-bicyclo[3.2.1]oct-3-yl}-1H-benzoimidazole

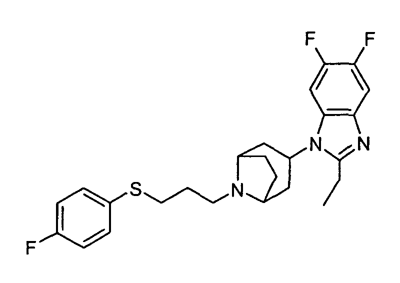

### METHOD OF TREATMENT

Accordingly, the present invention is directed to compounds which are useful in the prevention and/or treatment of a wide variety of inflammatory, infectious, and immunoregulatory disorders and diseases, including asthma and allergic diseases, infection by pathogenic microbes (which, by definition, includes viruses), as well as autoimmune pathologies such as the rheumatoid arthritis and atherosclerosis.

For example, an instant compound which inhibits one or more functions of a mammalian chemokine receptor (e. g., a human chemokine receptor) may be administered to inhibit (i. e., reduce or prevent) inflammation or infectious disease. As a result, one or more inflammatory process, such as leukocyte emigration, adhesion, chemotaxis, exocytosis (e. g., of enzymes, histamine) or inflammatory mediator release, is inhibited. For example, eosinophilic infiltration to inflammatory sites (e. g., in asthma or allergic rhinitis) can be inhibited according to the present method. In particular, the compound of the following examples has activity in blocking the migration of cells expressing the CCR-3 receptor using the appropriate chemokines in the aforementioned assays.

Similarly, an instant compound which promotes one or more functions of the mammalian chemokine receptor (e. g., a human chemokine) as administered to stimulate (induce or enhance) an immune or inflammatory response, such as leukocyte emigration, adhesion, chemotaxis, exocytosis (e. g., of enzymes, histamine) or inflammatory mediator release, resulting in the beneficial stimulation of inflammatory processes. For example, eosinophils can be recruited to combat parasitic infections. In addition, treatment of the aforementioned inflammatory, allergic and autoimmune diseases can also be contemplated for an instant compound which promotes one or more functions of the mammalian chemokine receptor if one contemplates the delivery of sufficient compound to cause the loss of receptor expression on cells through the induction of chemokine receptor internalization or the delivery of compound in a manner that results in the misdirection of the migration of cells.

In addition to primates, such as humans, a variety of other mammals can be treated according to the method of the present invention. For instance, mammals, including but not limited to, cows, sheep, goats, horses, dogs, cats, guinea pigs, rats or other bovine, ovine, equine, canine, feline, rodent or murine species can be treated. However, the method can also be practiced in other species, such as avian species. The subject treated in the methods above is a mammal, male or female, in whom modulation of chemokine receptor activity is desired. "Modulation" as used herein is intended to encompass antagonism, agonism, partial antagonism and/or partial agonism.

Diseases or conditions of human or other species which can be treated with inhibitors of chemokine receptor function, include, but are not limited to: inflammatory or allergic diseases and conditions, including respiratory allergic diseases such as asthma, allergic rhinitis, hypersensitivity lung diseases, hypersensitivity pneumonitis, eosinophilic cellulitis (e. g., Well's syndrome), eosinophilic pneumonias (e. g., Loeffler's syndrome, chronic eosinophilic pneumonia), eosinophilic fasciitis (e. g., Shulman's syndrome), delayed-type hypersensitivity, interstitial lung diseases (ILD) (e. g., idiopathic pulmonary fibrosis, or ILD associated with rheumatoid arthritis, systemic lupus erythematosus, ankylosing spondylitis, systemic sclerosis, Sjogren's syndrome, polymyositis or dermatomyositis); systemic anaphylaxis or hypersensitivity responses, drug allergies (e. g., to penicillin, cephalosporins), eosinophilia-myalgia syndrome due to the ingestion of contaminated tryptophan, insect sting allergies; autoimmune diseases, such as rheumatoid arthritis, psoriatic arthritis, multiple sclerosis, systemic lupus erythematosus, myasthenia gravis, juvenile onset diabetes; glomerulonephritis, autoimmune thyroiditis, Behcet's disease; graft rejection (e. g., in transplantation), including allograft rejection or graftversus-host disease; inflammatory bowel diseases, such as Crohn's disease and ulcerative colitis; spondyloarthropathies; scleroderma; psoriasis (including Tcell mediated psoriasis) and inflammatory dermatoses such as an dermatitis, eczema, atopic dermatitis, allergic contact dermatitis, urticaria; vasculitis (e. g., necrotizing, cutaneous, and hypersensitivity vasculitis); eosinophilic myositis, eosinophilic fasciitis; cancers with leukocyte infiltration of the skin or organs. Other diseases or conditions in which undesirable inflammatory responses are to be inhibited can be treated, including, but not limited to, reperfusion injury, atherosclerosis, certain hematologic malignancies, cytokine-induced toxicity (e. g., septic shock, endotoxic shock), polymyositis, dermatomyositis. Infectious diseases or conditions of human or other species which can be treated with inhibitors of chemokine receptor function, include, but are not limited to, HIV.

Diseases or conditions of humans or other species which can be treated with promoters of chemokine receptor function, include, but are not limited to: immunosuppression, such as that in individuals with immunodeficiency syndromes such as AIDS or other viral infections, individuals undergoing radiation therapy, chemotherapy, therapy for autoimmune disease or drug therapy (e. g., corticosteroid therapy), which causes immunosuppression; immunosuppression due to congenital deficiency in receptor function or other causes; and infections diseases, such as parasitic diseases, including, but not limited to helminth infections, such as nematodes (round worms); (Trichuriasis, Enterobiasis, Ascariasis, Hookworm, Strongyloidiasis, Trichinosis, filariasis) ; trematodes (flukes) (Schistosomiasis, Clonorchiasis), cestodes (tape worms) (Echinococcosis, Taeniasis saginata, Cysticercosis); visceral worms, visceral larva migraines (e. g., Toxocara), eosinophilic gastroenteritis (e. g., Anisaki sp., Phocanema sp.), cutaneous larva migraines (Ancylostona braziliense, Ancylostoma caninum). The compounds of the present invention are accordingly useful in the prevention and treatment of a wide variety of inflammatory, infectious and immunoregulatory disorders and diseases. In addition, treatment of the aforementioned inflammatory, allergic and autoimmune diseases can also be contemplated for promoters of chemokine receptor function if one contemplates the delivery of sufficient compound to cause the loss of receptor expression on cells through the induction of chemokine receptor internalization or delivery of compound in a manner that results in the misdirection of the migration of cells.

In another aspect, the instant invention may be used to evaluate the putative specific agonists or antagonists of a G protein coupled receptor. The present invention is directed to the use of these compounds in the preparation and execution of screening assays for compounds that modulate the activity of chemokine receptors. Furthermore, the compounds of this invention are useful in establishing or determining the binding site of other compounds to chemokine receptors, e. g., by competitive inhibition or as a reference in an assay to compare its known activity to a compound with an unknown activity. When developing new assays or protocols, compounds according to the present invention could be used to test their effectiveness.

Specifically, such compounds may be provided in a commercial kit, for example, for use in pharmaceutical research involving the aforementioned diseases. The compounds of the instant invention are also useful for the evaluation of putative specific modulators of the chemokine receptors. In addition, one could utilize compounds of this invention to examine the specificity of G protein coupled receptors that are not thought to be chemokine receptors, either by serving as examples of compounds which do not bind or as structural variants of compounds active on these receptors which may help define specific sites of interaction.

The CCR-3 receptor binding test is based on a K562 cell line (leukemia myelogenic blast cells ) transfected with the human chemokine receptor CCR-3 (hCCR-3-C1).
The cell membranes were prepared by disrupting the hCCR-3 transfected K562 cells by nitrogen decomposition and centrifugation at 40000 g , 4 °C for 1h. The membranes were re-suspended in the SPA incubation buffer without bovine serum albumin for storage in aliquots at -80 °C .

The CCR-3 receptor binding assay with the radioligand ¹²⁵jodine-eotaxin-1 was performed in a Scintillation Proximity Assay ( SPA ) design. Cell membranes of hCCR-3 Cl cells were diluted in suitable concentrations ( 0.5 -5 ug protein / well ) in 96 well microtiter plates ( 1450-401, Wallac).

The test incubation mixture comprising 60 µl of the membrane suspension, 80 µl of the Wheat Germ Agglutinin coated PVT beads (organic scintillator, Amersham Pharmacia biotech) in a concentration of 0,4 mg and 40 µl of radiolabelled ¹²⁵J rhEotaxin (Amersham, IM290) were incubated with 20 µl of the test compound (dissolved in DMSO dilutions ) for 2 hours. The SPA incubation buffer contained 25mM HEPES, 25mM MgCl₂ 6xH₂O, 1mM CaCl₂ 2xH₂O and 0,1% bovine serum albumin . Included were controls for specific binding ( no displacer added ) and non-specific binding by adding unlabelled rhEotaxin (R&D Systems) or a test compound. Bound radioactivity was determined by scintillation counter (Micro Beta "Trilux", Wallac).
Determination of affinity of test compounds ( dissociation constant Kᵢ ) was calculated by iterative fitting of experimental data using the law of mass action based program "easy sys" (Schittkowski, Num Math 68, 129-142 (1994)).

The utility of the compounds in accordance with the present invention as modulators of chemokine receptor activity may be demonstrated by methodology known in the art, such as the assays for CCR-2 and CCR-3 ligand binding, as disclosed by Ponath et al., J. Exp. Med., 183,2437-2448 (1996) and Uguccioni et al., J. Clin. Invest., 100,11371143 (1997). Cell lines for expressing the receptor of interest include those naturally expressing the chemokine receptor, such as EOL-3 or THP-1, those induced to express the chemokine receptor by the addition of chemical or protein agents, such as HL-60 or AML14.3D10 cells treated with, for example, butyric acid with interleukin-5 present, or a cell engineered to express a recombinant chemokine receptor, such as CHO or HEK-293. Finally, blood or tissue cells, for example human peripheral blood eosinophils, isolated using methods as described by Hansel et al., J. Immunol. Methods, 145,105-110 (1991), can be utilized in such assays. In particular, the compounds of the present invention have activity in binding to the CCR-3 receptor in the aforementioned assays. As used herein, "activity" is intended to mean a compound demonstrating an IC50 of 10 MM or lower in concentration when measured in the aforementioned assays. Such a result is indicative of the intrinsic activity of the compounds as modulators of chemokine receptor activity.

Binding constants Kᵢ are (Eotaxin at CCR-3-Rezeptor):

| Example | Kᵢ |
|---|---|
| 9 | 10 nM |
| 10 | 6.5 nM |
| 11 | 14 nM |

### PHARMACEUTICAL FORMS

The compounds are administered to a mammal in a therapeutically effective amount. By "therapeutically effective amount" it is meant an amount of a compound of formula 1 that, when administered alone or in combination with an additional therapeutic agent to a mammal, is effective to prevent or ameliorate diseases, wherein the activity of a CCR-3-receptor is involved, or the progression of this disease.

The compounds of this invention can be administered in such oral dosage forms as tablets, capsules (each of which includes sustained release or timed release formulations), pills, powders, granules, elixirs, tinctures, suspensions, syrups, and emulsions. They may also be administered in intravenous (bolus or infusion), intraperitoneal, subcutaneous, or intramuscular form, all using dosage forms well known to those of ordinary skill in the pharmaceutical arts. They can be administered alone, but generally will be administered with a pharmaceutical carrier selected on the basis of the chosen route of administration and standard pharmaceutical practice.

The dosage regimen for the compounds of the present invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. A physician or veterinarian can determine and prescribe the effective amount of the drug required to prevent, counter, or arrest the progress of the disorder.

By way of general guidance, the daily oral dosage of each active ingredient, when used for the indicated effects, will range between about 0.001 to 1000 mg/kg of body weight, preferably between about 0.01 to 100 mg/kg of body weight per day, and most preferably between about 1.0 to 20 mg/kg/day. Intravenously, the most preferred doses will range from about 1 to about 10 mg/kg/minute during a constant rate infusion. Compounds of this invention may be administered in a single daily dose, or the total daily dosage may be administered in divided doses of two, three, or four times daily.

Compounds of this invention can be administered in intranasal form via topical use of suitable intranasal vehicles, or via transdermale routes, using transdermale skin patches. When administered in the form of a transdermale delivery system, the dosage administration will, of course, be continuous rather than intermittent throughout the dosage regimen.

The compounds are typically administered in admixture with suitable pharmaceutical diluents, excipients, or carriers (collectively referred to herein as pharmaceutical carriers) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water, and the like. Moreover, when desired or necessary, suitable binders, lubricants, disintegrating agents, and colouring agents can also be incorporated into the mixture. Suitable binders include starch, gelatine, natural sugars such as glucose or beta-lactose, corn sweeteners, natural and synthetic gums such as acacia, tragacanth, or sodium alginate, carboxymethylcellulose, polyethylene glycol, waxes, and the like. Lubricants used in these dosage forms include sodium oleate, sodium stearate, magnesium stearate; sodium benzoate, sodium acetate, sodium chloride, and the like. Disintegrators include, without limitation, starch, methyl cellulose, agar, bentonite, xanthan gum, and the like.

The compounds of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidylcholines.

Compounds of the present invention may also be coupled with soluble polymers as targetable drug carriers. Such polymers can include polyvinylpyrrolidone, pyran copolymer, polyhydroxypropylmethacrylamide-phenol, polyhydroxyethylaspart-amidephenol, or polyethyleneoxidepolylysine substituted with palmitoyl residues.

Furthermore, the compounds of the present invention may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacylates, and cross linked or amphipathic block copolymers of hydro gels.

Dosage forms (pharmaceutical compositions) suitable for administration may contain from about 1 milligram to about 100 milligrams of active ingredient per dosage unit.

In these pharmaceutical compositions the active ingredient will ordinarily be present in an amount of about 0.5-95% by weight based on the total weight of the composition.

Gelatine capsules may contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain colouring and flavouring to increase patient acceptance.

In general, water, suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl-or propyl-paraben, and chlorobutanol.

Suitable pharmaceutical carriers are described in Reminqton's Pharmaceutical Sciences, Mack Publishing Company, a standard reference text in this field.

Where two or more of the foregoing second therapeutic agents are administered with the compound of formula 1, generally the amount of each component in a typical daily dosage and typical dosage form may be reduced relative to the usual dosage of the agent when administered alone, in view of the additive or synergistic effect of the therapeutic agents when administered in combination.

Particularly when provided as a single dosage unit, the potential exists for a chemical interaction between the combined active ingredients. For this reason, when the compound of formula 1 and a second therapeutic agent are combined in a single dosage unit they are formulated such that although the active ingredients are combined in a single dosage unit, the physical contact between the active ingredients is minimized (that is, reduced). For example, one active ingredient may be enteric coated. By enteric coating one of the active ingredients, it is possible not only to minimize the contact between the combined active ingredients, but also, it is possible to control the release of one of these components in the gastrointestinal tract such that one of these components is not released in the stomach but rather is released in the intestines. One of the active ingredients may also be coated with a material which effects a sustained-release throughout the gastrointestinal tract and also serves to minimize physical contact between the combined active ingredients.

Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a low viscosity grade of hydroxypropyl methylcellulose (HPMC) or other appropriate materials as known in the art, in order to further separate the active components. The polymer coating serves to form an additional barrier to interaction with the other component.

These as well as other ways of minimizing contact between the components of combination products of the present invention, whether administered in a single dosage form or administered in separate forms but at the same time by the same manner, will be readily apparent to those skilled in the art, once armed with the present disclosure.

## Claims

1. Use of a compound of general formula 1 for manufacturing a medicament for the prevention and/or treatment of diseases wherein the activity of a CCR-3-receptor is involved. 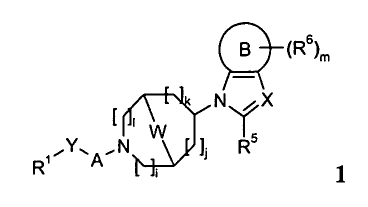 wherein
R¹ is aryl, het or a annelated species thereof, wherein het is a heterocyclic ring and the annelated species comprises aryl-het-, het-aryl- or het-het-annelations, each of said aryl or het may be substituted with one, two or three R²;
R² are each independently -C₁₋₆-alkyl, -C₃₋₆-cycloalkyl, -C₁₋₆-haloalkyl, -C₁₋₆-aralkyl, halogen, -CN, -COOR³, -COR³, -CONR³R⁴, -NR³R⁴, -NR³COR⁴, -NR³SO₂R⁴, -OR³, -NO₂, -SR³, -SOR³, -SO₂R³ or -SO₂NR³R⁴;
R³ is H, -C₁₋₆-alkyl, -C₃₋₈-cycloalkyl or (-C₃₋₈-cycloalkyl)-C₁₋₆-alkyl;
R⁴ is H, -C₁₋₆-alkyl, -C₃₋₈-cycloalkyl or (-C₃₋₈-cycloalkyl)-C₁₋₆-alkyl or
R³ and R⁴ together with the interjacent nitrogen atom or -N-SO₂- group form an optionally substituted nitrogen containing heterocyclic 3 to 8 membered ring.
R⁵ is -C₁₋₆-alkyl, -C₁₋₆-alkoxy, -C₁₋₆-acyloxy, -C₁₋₆-aralkyl, -C₃₋₆-cycloalkyl, (-C₃₋₆-cycloalkyl)-C₁₋₆-alkyl, -C₁₋₆-haloalkyl, -C₁₋₆-haloalkoxy, -C₁₋₆-haloalkylthio, -C₁₋₆-thioalkyl, halogen, -NO₂ -OH, -CN;
R⁶ are each independently -C₁₋₆-alkyl, -C₁₋₆-alkoxy, -C₁₋₆-acyloxy, -C₁₋₆-aralkyl, -C₃₋₆-cycloalkyl,-C₁₋₆-haloalkyl, -C₁₋₆-thioalkyl, halogen, -OR³, -SR³, -CN, -NO₂-COOR³, -COR³, -CONR³R⁴, -NR³R⁴, -NR³COR⁴, -NR³SO₂R⁴, -SOR³, -SO₂R³, -SO₂NR³R⁴, aryl or het;
A is -C₂₋₈-alkylene, optionally substituted with halogen or -OH;
B is aryl or het;
X is N, CH or CR⁶;
Y is -CF₂-, -NR⁴-, -O-, -S(O)ₙ-;
W is -C₁₋₃-alkylene, -CH₂-Z-CH₂-;
Z is -O-, -S-, -NR⁴-;
i, j, k, l are each independently 0, 1 or 2, wherein 2≤ i+ j + k + 1 ≤ 4;
n is 0, 1 or 2;
m is 0, 1, 2, 3 or 4.

2. A compound of general formula **1a,** 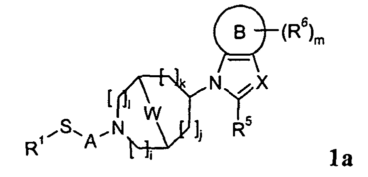 wherein R¹, R⁵, R⁶, A, B, X, W, Z, i, j, k, l and m are defined as in claim 1.

3. A compound of general formula **1b**, 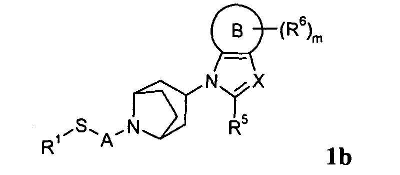 and R¹, R⁵, R⁶, A, B, X, Y and m are defined as in claim 1.

4. A compound according to claims 2 or 3, wherein
R¹ is aryl or het, both optionally substituted with one, two or three R² and
B is phenyl.

5. A compound according to claims 2-4, wherein
R¹ is phenyl, optionally substituted with one, two or three R² and
R⁵ is -C₁₋₆-acyloxy, -C₁₋₆-aralkyl, -C₃₋₆-cycloalkyl, (-C₃₋₆-cycloalkyl)-C₁₋₆-alkyl, -C₂₋₆-haloalkyl or -C₁₋₆-thioalkyl.

6. A compound according to claims 2-5, wherein
A is -CH₂-CH₂-CH₂-.

7. A compound of general formula **1c**, 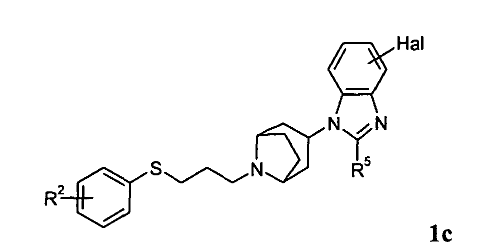 wherein
R⁵ is defined as in claim 5 and
R² is -CF₃ or halogen;
Hal is halogen.

8. A compound according to claims 2-7, wherein
R⁵ is -C₂₋₆-haloalkyl, -C₃₋₆-cycloalkyl.

9. A compound according to claims 2-6, wherein
R¹ is phenyl, optionally substituted with one, two or three R² and
m is 2, 3 or 4.

10. A compound according to claims 2-6 or 9, wherein
X is N.

11. A compound of general formula **1d**, 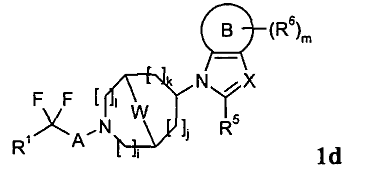 wherein R¹, R⁴, R⁵, R⁶, A, B, X, W, Z, i, j, k, l and m are defined as in claim 1.

12. A compound of general formula **1e,** 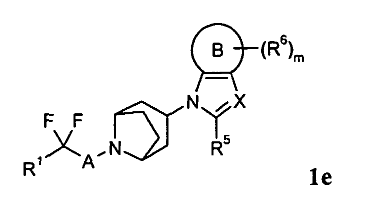 wherein R¹, R⁵, R⁶, A, B, X, Y and m are defined as in claim 1.

13. A compound according to claims 11 or 12, wherein
R¹ is aryl or het, both optionally substituted with up to three R² and
B is phenyl.

14. A compound according to claims 11-13, wherein
R¹ is phenyl, optionally substituted with one, two or three R².

15. A compound according to claims 11-14, wherein
A is -CH₂-CH₂-CH₂-.

16. A compound according to claims 11-15, wherein
X is N.

17. Pharmaceutical composition, **characterised in that** it contains one or more compounds of formula **1** according to one of claims 2-16.
